# EUROPÄISCHE PATENTANMELDUNG

(11) **EP 0 134 392 A1**
(43) Veröffentlichungstag der Anmeldung: **20.03.1985**
(21) Anmeldenummer: 84103638.7
(22) Anmeldetag: 03.04.1984
(51) Int. Cl.: C07C 99/00, C07C 101/18, C07C 101/22, C07C 101/28, C07D 295/14, C07D 211/34, C07D 207/16, C07C 143/68

(54) **Verfahren zur praktisch stereospezifischen Herstellung von optisch aktiven alpha-Aminocarbonsäureestern**

(30) Priorität: 11.08.1983 DE 3328986
(71) Anmelder: Degussa Aktiengesellschaft, D-60311 Frankfurt (DE)
(72) Erfinder: Drauz, Karlheinz, Dr. Dipl.-Chem., D-6463 Freigericht 1 (DE); Burkard, Ulrike, Dipl.-Chem., D-7000 Stuttgart 1 (DE); Effenberger, Franz, Prof. Dr., D-7000 Stuttgart 70 (DE)

(57) **Zusammenfassung**

Es wird ein Verfahren zur praktisch stereospezifischen Herstellung von bestimmten optisch aktiven a-Aminocarbonsäureestern beschrieben, bei welchem O-Perfluoralkylsulfonyl-hydroxycarbonsäureester in einem inerten Lösungsmittel bei einer Temperatur zwischen -80 und +50 °C mit Aminoverbindungen unter Einhaltung bestimmter Molmengen umgesetzt wird.

## Beschreibung

Die Erfindung betrifft ein Verfahren zur praktisch stereospezifischen Herstellung von optisch aktiven α-Aminocarbonsäureestern der allgemeinen Formel 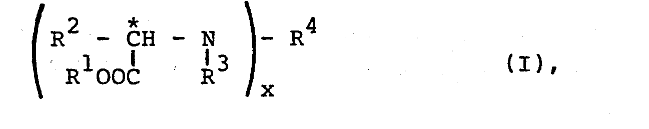 in der
^{*} ein Asymmetriezentrum,
x die Zahl 1 oder 2,
R¹ einen Methyl-, Ethyl- oder Benzylrest,
R² einen geradkettigen oder verzweigten, unsubstituierten oder ein- oder mehrfach und in beliebiger Stellung durch Fluor, Chlor, Brom, eine (C₁ bis C₈)-Alkoxygruppe, eine Methoxy-, Ethoxy- oder Benzoxycarbonylgruppe oder einen unsubstituierten oder 1,2 oder 3 Substituenten tragenden Phenylrest substituierten (C₁ bis C₈)-Alkylrest oder einen unsubstituierten oder 1,2 oder 3 Substituenten tragenden Phenylrest und
_{R}³ Wasserstoff, einen geradkettigen oder verzweigten, unsubstituierten oder ein- oder mehrfach und in beliebiger Stellung durch Fluor, Chlor, Brom, eine Cyanogruppe, eine Hydroxylgruppe, eine (C₁ bis C₈)-Alkoxygruppe, eine Methoxy-, Ethoxy- oder Benzoxycarbonylgruppe, eine Di-(C₁ bis C₈)-alkylaminogruppe, einen unsubstituierten oder 1,2 oder 3 Substituenten tragenden Phenylrest oder einen unsubstituierten oder 1 bis 6 Substituenten tragenden Naphthylrest substituierten (C₁ bis C12)-Alkylrest oder einen unsubstituierten oder 1,2 oder 3 Substituenten tragenden Phenylrest oder einen unsubstituierten oder 1 bis 6 Substituenten tragenden Naphthylrest bedeuten,
für x = 1 _{R}⁴ unabhängig von R³ mit Ausnahme von Wasserstoff eine der für R³ angegebenen Bedeutungen hat oder zusammen mit R³ und dem Stickstoffatom einen gesättigten oder ungesättigten Ring mit 4 bis 7 Ringgliedern bildet, der 0 bis 2 weitere Heteroatome aus der Gruppe Stickstoff, Schwefel oder Sauerstoff aufweist und unsubstituiert oder ein- oder mehrfach durch die oben definierten Substituenten R² oder R³ substituiert ist, oder für x = 2 R⁴ einen geradkettigen oder verzweigten Alkylenrest mit 1 bis 12 Kohlenstoffatomen darstellt.

Optisch aktive α-Aminocarbonsäureester der allgemeinen Formel (I) sind wichtige Zwischenprodukte bei der Herstellung von Wirkstoffen für den Pflanzenschutz und die ^{p}harmazeu- tische Anwendung. Sie weisen an dem α-Kohlenstoffatom, das die substituierte Aminogruppe trägt, ein Asymmetriezentrum auf. Untersuchungen haben gezeigt, daß in vielen Fällen das eine Enantiomere der Endprodukte wirksamer ist als das andere oder das Racemat. Deshalb wurden in der Vergangenheit schon erhebliche Bemühungen unternommen, eine möglichst stereoselektive Herstellung der optisch aktiven Zwischenprodukte zu erreichen.

So ist beispielsweise aus der DE-OS 30 37 159 ein Verfahren zur Herstellung von optisch aktiven 2-Anilinopropionsäureestern bekannt, bei dem optisch aktive Milchsäureestersulfonate, insbesondere Mesylate oder Tosylate, mit substituierten Anilinen umgesetzt werden. Bei diesem bekannten Verfahren ist es zur Erzielung einer annehmbaren chemischen Ausbeute erforderlich, recht drastische Reaktionsbedingungen anzuwenden, insbesondere relativ hohe Temperaturen und lange Reaktionszeiten. Außerdem muß das substituierte Anilin in beträchtlichem Überschuß eingesetzt werden. Die erforderlichen drastischen Reaktionsbedingungen wirken sich jedoch ungünstig auf die erzielbare optische Reinheit der gebildeten 2-Anilinopropionsäureester aus, die infolge teilweiser Racemisierung nur zwischen 75 und 90 % der Theorie beträgt.

Das erfindungsgemäße Verfahren ist nun dadurch gekennzeichnet, daß man einen 0-Perfluoralkylsulfonyl-hydroxycarbonsäureester der allgemeinen Formel 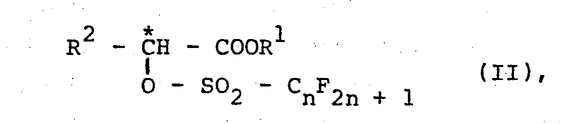 in der *, R¹ und _{R}² wieder die bereits angegebenen Bedeutung haben und n eine ganze Zahl von 1 bis 6 bedeutet, in einem inerten Lösungsmittel bei einer Temperatur zwischen -80 und +50 °C mit einer Aminoverbindung der allgemeinen Formel

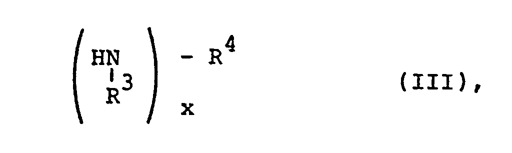

in der ₓ, R 3 und R⁴ wieder die bereits angegebene Bedeutung haben, umsetzt, wobei die Aminoverbindung der allgemeinen Formel (III), jeweils bezogen auf den eingesetzten 0-_{P}er- fluoralkylsulfonyl-hydroxycarbonsäureester der allgemeinen Formel (II), für x = 1 in der 2- bis 2,5-fachen Molmenge und für x = 2 in der 1- bis 1,25-fachen Molmenge eingesetzt wird.

Vorzugsweise wird die Umsetzung zwischen dem 0-Perfluoralkylsulfonyl-hydroxycarbonsäureester der allgemeinen Formel (II) mit der Aminoverbindung der allgemeinen Formel (III) bei einer Temperatur zwischen -40 und +25 C vorgenommen.

Der wesentliche Vorteil des erfindungsgemäße Verfahrens gegenüber dem aus der DE-OS 30 37 159 bekannten, besteht darin, daß es sich bei sehr viel niedrigeren Temperaturen überraschend glatt durchführen läßt, wodurch die Gefahr einer teilweisen Racemisierung deutlich vermindert wird. Gleichzeitig sind trotz der niedrigeren Reaktionstemperaturen zur Erzielung von vergleichbaren chemischen Ausbeuten im allgemeinen nur relativ kurze Reaktionszeiten erforderlich.

Unter den beim erfindungsgemäßen Verfahren einzusetzenden 0-Perfluoralkylsulfonyl-hydroxycarbonsäureestern der allgemeinen Formel (II) werden die Perfluor-n-butyl- und insbesondere die Perfluormethylsulfonyl-Derivate besonders bevorzugt. Sofern der Substituent R² einen Phenylrest darstellt oder enthält, kann dieser unsubstituiert sein oder 1,2 oder 3 Substituenten tragen, die in beliebiger Stellung zueinander angeordnet sein können. Beispielsweise kann der Phenylrest durch Fluor, Chlor, Brom, (C₁ bis C4)-Alkylreste, (Cₗ bis C₂)-Alkoxygruppen oder Di-(C₁ bis C₈)-alkylaminogruppen substituiert sein.

Die 0-Perfluoralkylsulfonyl-hydroxycarbonsäureester der allgemeinen Formel (II) können durch Umsetzung der Chloride, Bromide oder Anhydride entsprechender Perfluoralkylsulfonsäuren mit entsprechenden optisch aktiven α-Hydroxycarbonsäureestern in einem inerten Lösungsmittel und in Gegenwart eines Säureacceptors bei einer Temperatur zwischen -80 und +50 °C, vorzugsweise zwischen -70 und 0 °C, hergestellt werden. Als inerte Lösungsmittel kommen beispielsweise infrage Halogenkohlenwasserstoffe, wie Chloroform, Tetrachlorkohlenstoff, 1,2-Dichlorethan, Trichlorethylen und insbesondere Dichlormethan. Als Säureacceptoren können beispielsweise tertiäre Amine, wie Triethylamin, Tri-n-butylamin oder Pyridin dienen. Die optisch aktiven α-Hydroxycarbonsäureester können in bekannter Weise aus natürlich vorkommenden oder in technischem Maßstab leicht, z.B. durch biotechnologische Verfahren oder aus den entsprechenden optisch aktiven α-Aminocarbonsäuren, herstellbaren α-Hydroxycarbonsäuren gewonnen werden.

Die Reingewinnung der 0-Perfluoralkylsulfonyl-hydroxycarbonsäureester der allgemeinen Formel (II) kann in der Weise erfolgen, daß nach der Herstellung das inerte Lösungsmittel abdestilliert und der Rückstand in einem unpolaren Kohlenwässerstoff, z.B. n-Pentan, aufgenommen wird. Die dabei ungelöst bleibenden Salze werden abfiltriert, das Lösungsmittel abgedampft und der Rückstand durch fraktionierte Destillation oder Chromatographie weiter gereinigt. Die 0-Perfluoralkylsulfonyl-hydroxycarbonsäureester sind dann im allgemeinen analysenrein.

Für die Praxis besonders wichtige 0-Perfluoralkylsulfonyl-hydroxycarbonsäureester der allgemeinen Formel (II) sind der (S)- oder (R)-O-Trifluormethylsulfonyl-milchsäureme- thyl-, -ethyl- oder -benzylester, der (S)- oder (R)-0-Trifluormethylsulfonyl-äpfelsäuremethyl-, -ethyl- oder -benzylester und der (S)- oder (R)-O-Trifluormethylsulfonyl- phenylmilchsäuremethyl-, -ethyl- oder -benzylester.

Bei den beim erfindungsgemäßen Verfahren einzusetzenden Aminoverbindungen der allgemeinen Formel (III) kann es sich um Monoaminoverbindungen (x = 1) oder um Diaminoverbindungen (x = 2) handeln. Sofern die Substituenten _{R}³ und/oder R⁴ einen Phenylrest oder Naphthylrest enthalten, können diese Reste unsubstituiert sein oder der Phenylrest 1, 2 oder 3 Substituenten und der Naphthylrest 1 bis 6 Substituenten tragen, die in beliebiger Stellung zueinander angeordnet sein können. Beispielsweise kann der Phenylrest oder der Naphthylrest durch Fluor, Chlor, Brom, (C₁ bis C4)-Alkylreste, (C₁ bis C₂)-Alkoxygruppen oder Di-(C₁ bis C₈)-alkylaminogruppen substituiert sein. Die Naphthylreste können in der 1- oder in der 2-Stellung gebunden sein.

Bei den Aminoverbindungen der allgemeinen Formel (III) handelt es sich in vielen Fällen um leicht zugängliche handelsübliche Verbindungen. In allen anderen Fällen können sie in für die Herstellung von Aminoverbindungen bekannter Weise gewonnen werden. Monoaminoverbindungen werden in der 2- bis 2,5-fachen Molmenge, Diaminoverbindungen in der 1- bis 1,25-fachen Molmenge, jeweils bezogen auf den eingesetzten 0-Perfluoralkylsulfonyl-hydroxycarbonsäureester der allgemeinen Formel (II), eingesetzt. Im allgemeinen reicht schon ein Überschuß an Aminoverbindung der allgemeinen Formel (III) aus, der gerade in der Lage ist, die austretende 0-Perfluoralkylsulfonsäure als Säureacceptor zu binden.

Für die Praxis besonders wichtige Aminoverbindungen der allgemeinen Formel (III) sind unter den Monoaminoverbindungen unsubstituierte oder substituierte Mono- und Dialkylamine, wie Ethanolamin, tert. Butylamin, n-Hexylamin oder Diethylamin; unsubstituierte oder substituierte aromatische Amine, wie Anilin, 2-Chloranilin, 2₋Ethylanilin, 2,6-Dimethylanilin, 3-Hydroxyanilin, 3-Methoxyanilin, 3,4-Dichloranilin, 4-Chloranilin oder α-Naphthylamin; Aralkylamine, wie Benzylamin oder α-Methylbenzylamin; Alkylaryl-oder Alkylalkarylamine, wie N-Methylanilin oder N-Benzyl-N-Methylamin; Heterocyclen, wie Pyrrolidin; oder Aminocarbonsäureester, die gegebenenfalls ihrerseits bereits optisch aktiv sein können, wie e-Aminocapronsäuremethylester, (R)- und (S)-Prolinmethylester oder (R)- und (S)-Phenylalaninmethylester. Unter den Diaminoverbindungen kommen insbesondere geradkettige oder verzweigte α,ω-Diamine mit 2 bis 6 Kohlenstoffatomen in Frage, wie Ethylendiamin oder Hexamethylendiamin-1,6.

Weitere Beispiele sind Alanin-, Glycin-, Leucin-, Asparagin-, Glutamin-, Tyrosin-, α-Z-Lysin- und ε-Z-Lysinester.

Die Umsetzung zwischen dem 0-Perfluoralkylsulfonyl-hydroxycarbonsäureester der allgemeinen Formel (II) mit der Aminoverbindung der allgemeinen Formel (III) wird in einem inerten Lösungsmittel vorgenommen. Geeignete Lösungsmittel sind beispielsweise Halogenkohlenwasserstoffe, wie _{D}i-chlormethan oder Tetrachlorkohlenstoff; Ether, wie _{D}i-ethylether, Diisopropylether, Methyl-tert. butylether; aliphatische oder aromatische Kohlenwasserstoffe, wie n-Pentan, n-Hexan, Benzol oder Toluol; Cyclohexan, Acetonitril oder Aceton.

Die Umsetzung wird zweckmäßigerweise so vorgenommen, daß man den einen Reaktionsteilnehmer vorlegt und den anderen langsam zugibt. Dabei spielt es häufig keine Rolle, ob der 0-Perfluoralkylsulfonyl-hydroxycarbonsäureester der allgemeinen Formel (II) vorgelegt und die Aminoverbindung der allgemeinen Formel (III) zugegeben wird oder umgekehrt. Nach beendeter Zugabe des zweiten Reaktionsteilnehmers ist die Umsetzung in vielen Fällen ebenfalls schon beendet, in anderen Fällen ist eine angemessene Nachreaktionszeit für die Verbesserung der chemischen Ausbeute von Vorteil. Auf jeden Fall ist die gesamte Reaktionszeit absolut unkritisch, weil selbst bei Reaktionszeiten von etwa 50 Stunden nicht mit einer Racemisierung des gebildeten «-Aminocarbonsäureesters der allgemeinen Formel (I) gerechnet werden muß.

Nach beendeter Umsetzung werden zweckmäßigerweise zunächst die gebildeten Ammoniumsalze durch Abfiltrieren oder Abzentrifugieren, sofern das verwendete Lösungsmittel mit Wasser nicht mischbar ist, gegebenenfalls auch durch Auswaschen mit Wasser abgetrennt. Anschließend wird dann das LösUngsmittel durch Destillation entfernt.

Gegebenenfalls kann aber auch zuerst das Lösungsmittel abdestilliert werden und dann die Abtrennung der gebildeten Ammoniumsalze erfolgen. Bei dieser Variante kann es vorteilhaft sein, zur vollständigen Abscheidung der Ammoniumsalze ein unpolares Lösungsmittel, wie n-Pentan, n-Hexan, n-Heptan oder Cyclohexan, zuzusetzen, das dann wieder abgedampft wird.

Die von Salzen und Lösungsmitteln befreiten Rückstände werden dann entweder durch fraktionierte Destillation unter vermindertem Druck oder, sofern sie nicht destillierbar sind, durch Chromatographie über eine Kieselgel-Säule weiter gereinigt. Auf diese Weise lassen sich die optisch aktiven /5-Aminocarbonsäureester der allgemeinen Formel (I) in hoher Ausbeute analysenrein gewinnen. Sie haben im allgemeinen eine enantiomere bzw. diastereomere Reinheit von über 98 %, bezogen auf die optische Reinheit der eingesetzten 0-Perfluoralkylsulfonyl-hydroxycarbonsäureester der allgemeinen Formel (II) und, sofern diese ihrerseits optisch aktiv sind, der Aminoverbindungen der allgemeinen Formel (III). In diesem letzteren Falle kann die optische Reinheit durch gaschromatograpnische Bestimmung des Diastereomerenverhältnisses im gebildeten Produkt ermittelt werden.

Im folgenden wird zunächst die Herstellung einiger 0-Perfluoralkylsulfonyl-hydroxycarbonsäureester der allgemeinen Formel (II) beschrieben, die in den späteren Beispielen Verwendung finden sollen:

### 1. (S)-0-Trifluormethylsulfonyl-milchsäuremethylester

Zu einer Lösung von 22,57 g (80,0 mmol) Trifluormethansulfonsäureanhydrid in 80 ml Dichlormethan wurden bei 0 °C 8,33 g (80,0 mmol) (S)-Milchsäuremethylester und 6,33 g (80,0 mmol) Pyridin in 80 ml Dichlormethan innerhalb von 60 Minuten zugetropft. Nach Erwärmen auf 25 °C wurde das Dichlormethan bei einem Druck von 20 mbar abgedampft und der Rückstand in 100 ml n-Pentan aufgenommen. Nach Absaugen der ungelösten Salze und Entfernen des n₋Pentans wurde unter vermindertem Druck destilliert. Bei 68 °C/16 mbar gingen 13,42 g (71 % der Theorie) an (S)-0-Trifluormethylsulfonyl-milchsäuremethylester über.

Elementaranalyse:

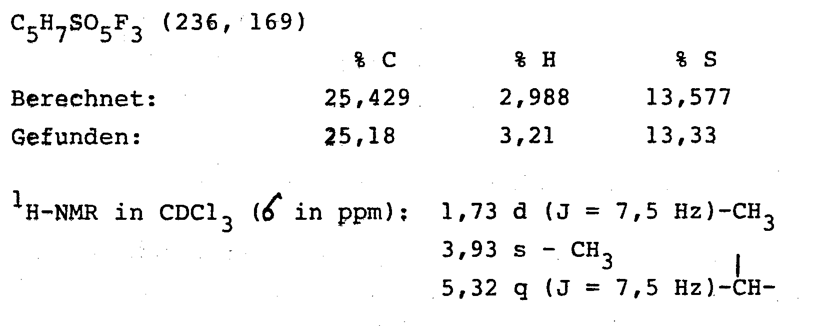

### 2. (S)-0-Trifluormethylsulfonyl-milchsäureethylester

Zu einer Lösung von 7,05 g (25,0 mmol) Trifluormethansulfonsäureanhydrid in 50 ml Tetrachlorkohlenstoff wurden bei 0 °C 2,95 g (25,0 mmol) (S)-Milchsäureethylester und 5,67 g (25,0 mmol) Di-(2₋methylpropyl)-2,4-dimethylpentyl-(3)-amin in 50 ml Tetrachlorkohlenstoff innerhalb von 45 Minuten zugetropft. Danach wurde noch 30 Minuten lang ohne Kühlung nachgerührt, anschließend das Lösungsmittel unter vermindertem Druck abdestilliert und der Rückstand mit 50 ml n-Pentan versetzt. Die ungelösten Salze wurden abgesaugt, die n-Pentanphase mit 25 ml Eiswasser gewaschen und über Magnesiumsulfat getrocknet. Dann wurde das n-Pentan bei 27 mbar abgedampft und der Rückstand einer fraktionierten Destillation unterworfen. Bei 33 bis 35 °C/0,07 mbar gingen 6,0 g (95,9 % der Theorie) an (S)-0-Trifluormethylsulfonyl-milchsäureethylester über.

### Elementaranalyse:

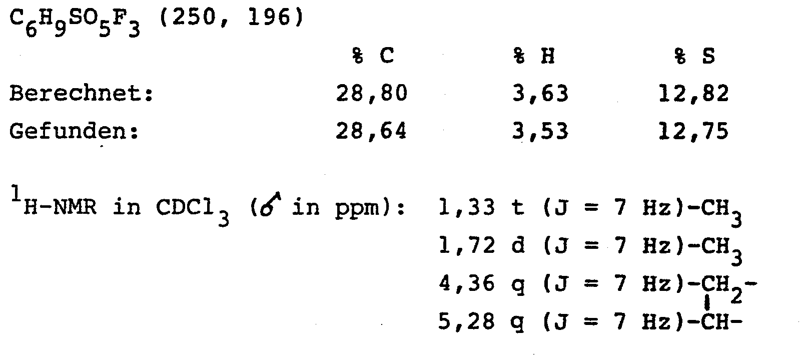

### 3. (S)-O-Trifluormethylsulfonyl-äpfelsäurediethylester

Zu einer Lösung von 26,45 g (94,0 mmol) Trifluormethansulfonsäureanhydrid in 100 ml Dichlormethan wurden bei -65 °C 17,83 g (94,0 mmol) (S)-Äpfelsäurediethylester und 7,44 g (94,0 mmol) Pyridin in 100 ml Dichlormethan innerhalb von 2,25 Stunden zugetropft. Nach Erwärmen auf 25 °C wurde das Dichlormethan unter vermindertem Druck entfernt und der Rückstand mit 200 ml n-Pentan versetzt. Die ungelösten Salze wurden abgesaugt und das n-Pentan unter vermindertem Druck abgezogen. Als Rückstand verblieben 27,11 g (89,5 % der Theorie) an (S)-0-Trifluormethylsulfonyl- äpfelsäurediethylester. Nach Umkristallisation aus n-Pentan lag der Schmelzpunkt bei 37,5 bis 38 °C. Elementaranalyse:

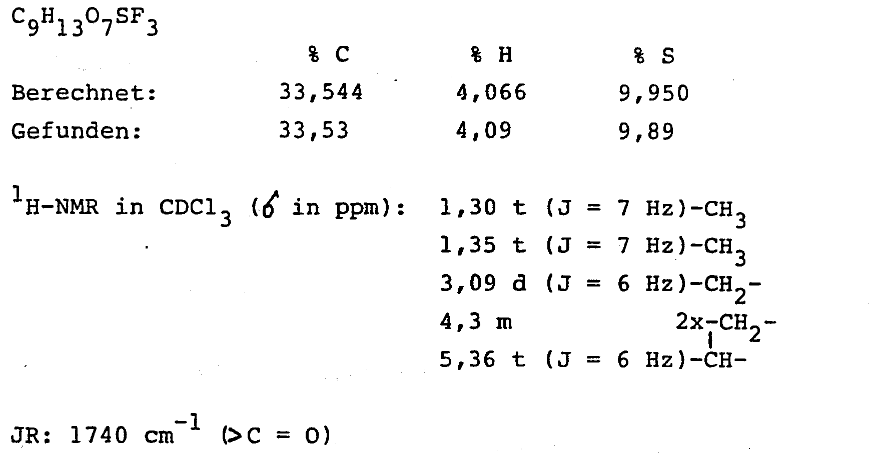

### .4. (S)-0-Trifluormethylsulfonyl-phenylmilchsäuremethyl- ester

Zu einer Lösung von 11,29 g (40,0 mmol) Trifluormethansulfonsäureanhydrid in 50 ml Dichlormethan wurde im Temperaturbereich zwischen -35 und -30 °C eine Lösung von 7,21 g (40,0 mmol) (S)-Phenylmilchsäuremethylester und 3,48 g (44,0 mmol) Pyridin in 80 ml Dichlormethan innerhalb von 45 Minuten zugetropft. Nach vierstündigem Nachrühren bei -30 °C und Erwärmen auf 25 C wurden die ausgefallenen Salze abfiltriert und das Lösungsmittel unter vermindertem Druck entfernt. Der Rückstand wurde anschließend über eine 20 cm hohe Kieselgel-Säule gereinigt (Laufmittel: Dichlormethan : n-Pentan = 1 : 1). Nach dem Eindampfen des Eluats und Entfernen der Lösungsmittelreste im Hochvakuum verblieben 10,21 g (82,0 % der Theorie) an (S)-0-Trifluormethylsulfonyl-phenylmilchsäuremethylester.

### Elementaranalyse:

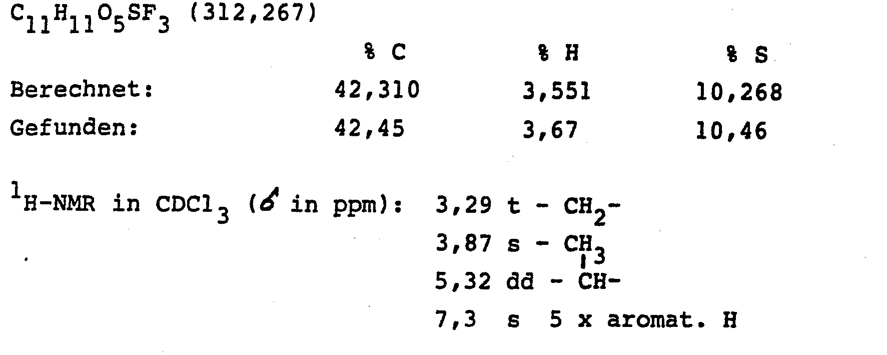

### 5. (S)-O-Trifluormethylsulfonyl-äpfelsäuredibenzylester

Zu einer Lösung von 7,05 g (25,0 mmol) Trifluormethansulfonsäureanhydrid in 50 ml Dichlormethan wurden bei -70 °C 7,86 g (25,0 mmol) (S)-Äpfelsäuredibenzylester und 2,14 g (27,0 mmol) Pyridin in 80 ml Dichlormethan innerhalb von 1 Stunde zugetropft. Nach einstündigem Nachrühren und Erwärmen auf Raumtemperatur wurden die ausgefallenen Salze abgesaugt und das Lösungsmittel unter vermindertem Druck entfernt. Der Rückstand wurde anschließend über eine 30 cm hohe Kieselgel-Säule gereinigt (Laufmittel: Dichlormethan). Nach dem Eindampfen des Eluats, gegen Ende im Hochvakuum, verblieben 9,85 g (88 % der Theorie) an (S)-O-Trifluormethylsulfonyl-äpfelsäuredibenzylester mit einem Schmelzpunkt von 39 bis 40 °C.

### Elementaranalyse:

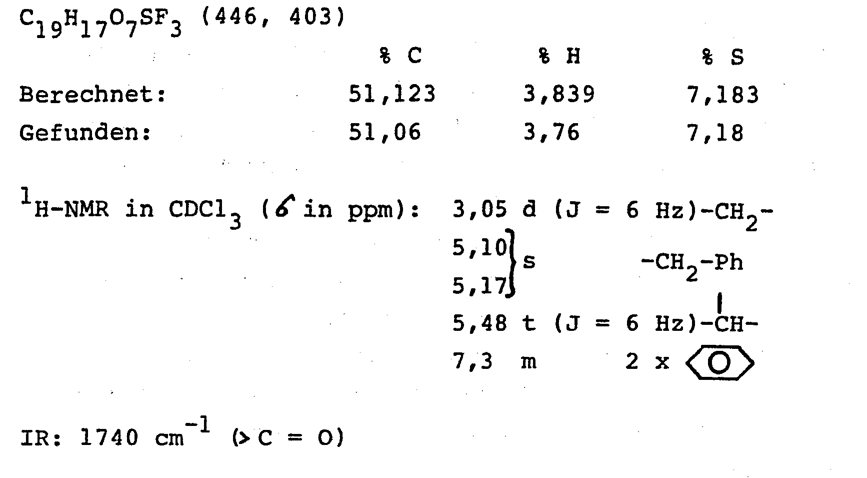

### 6. (R)-0-Trifluormethylsulfonyl-äpfelsäuredibenzylester

Analog wie vorstehend unter 5. beschrieben wurden 4,72 g (15,0 mmol) (R)-Äpfelsäuredibenzylester und 1,28 g (16,2 mmol) Pyridin in 40 ml Dichlormethan mit 4,23 g (15,0 mmol) Trifluormethansulfonsäureanhydrid in 30 ml Dichlormethan umgesetzt. Es wurden 6,20 g (93 % der Theorie) an (R)-O-Trifluormethylsulfonyl-äpfelsäuredibenzylester als farbloses Öl erhalten.

Die Erfindung wird durch die nachstehenden Beispiele näher erläutert:

### Beispiel 1:

Zu 24,24 g (200,0 mmol) 2,6-Dimethylanilin in 100 ml Dichlormethan wurde innerhalb von 20 Minuten bei 25 °C eine Lösung von 23,62 g (100,0 mmol) (S)-0-Trifluormethylsulfonyl-milchsäuremethylester in 100 ml Dichlormethan zugetropft. Nach 17-stündigem Nachrühren bei 25 ⁰C wurde das Lösungsmittel unter vermindertem Druck abdestilliert, der Rückstand in 300 ml n-Pentan aufgenommen, von den ungelösten Salzen abgesaugt, das n-Pentan abdestilliert und der ölige Rückstand unter vermindertem Druck fraktioniert destilliert. Bei 64 °C/0,006 mbar gingen 18,74 g (90,4 % der Theorie) an (R)-2-[(N-2,6-dimethylphenyl)-amino]-propion- säuremethylester über.

### Elementaranalyse:

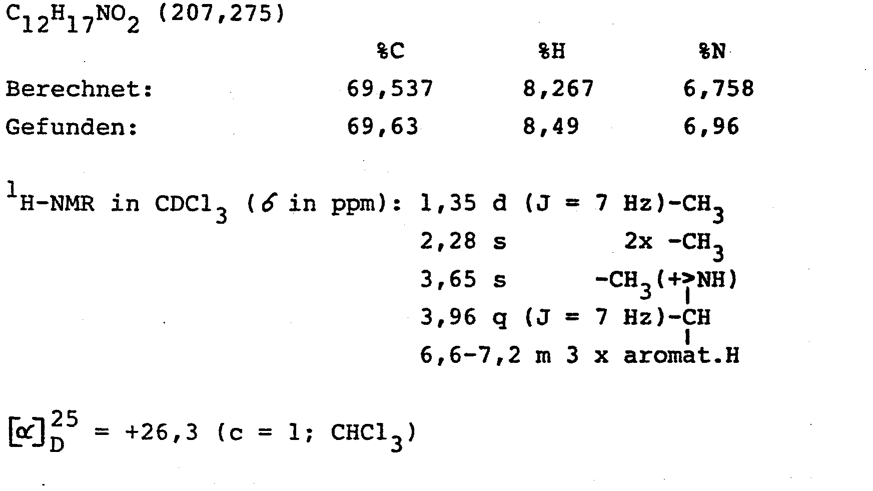

### Beispiel 2:

Zu 6,06 g (50,0 mmol) 2,6-Dimethylanilin in 40 ml Dichlormethan wurde innerhalb von 10 Minuten bei 25 °C eine Lösung von 6,25 g (25,0 mmol) (S)-0-Trifluormethylsulfonyl-milch- säureethylester in 40 ml Dichlormethan zugetropft. Nach 17- stündigem Nachrühren bei 25 °C wurde das Lösungsmittel unter vermindertem Druck abdestilliert, der Rückstand in 100 ml n-Pentan aufgenommen, von den ungelösten Salzen abgesaugt, das n-Pentan abdestilliert und er ölige Rückstand unter vermindertem Druck fraktioniert destilliert. Bei 74 °C/0,006 mbar gingen 5,02 g (90,7 % der Theorie) an (R)-2-[(N-2,6-dimethylphenyl)-amino]-propionsäureethylester über.

### Elementaranalyse:

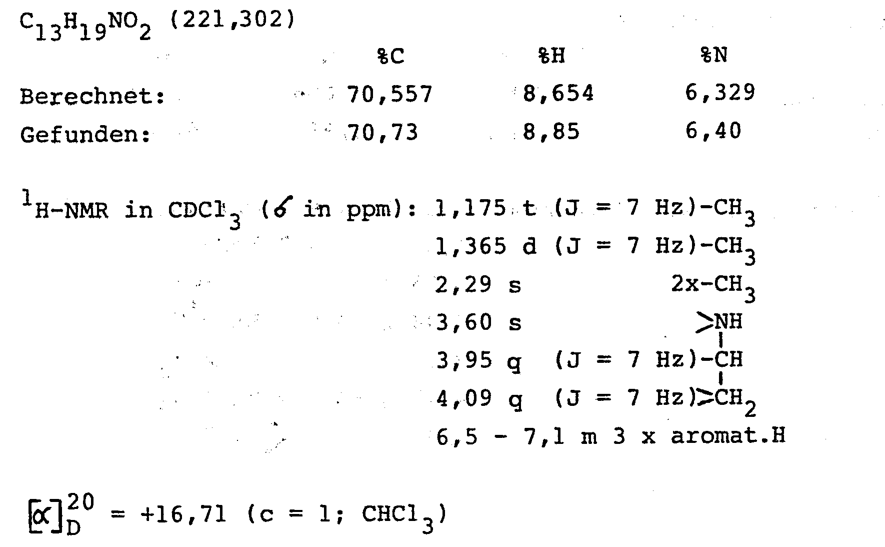

### Beispiel 3:

Zu 4,85 g (40,0 mmol) N-Benzyl-N-methylamin in 40 ml Dichlormethan wurde innerhalb von 30 Minuten bei 0 °C eine Lösung von 5,00 g (20,0 mmol) (S)-0-Trifluormethylsulfonyl-milchsäureethylester in 40 ml Dichlormethan zugetropft. Nach 1,5-stündigem Nachrühren bei 25 °C wurde von den ausgefallenen Salzen abfiltriert und das Lösungsmittel abdestilliert. Der Rückstand wurde in n-Pentan aufgenommen, die noch ausgefallenen Salzreste abfiltriert, das n-Pentan abdestilliert und die verbliebenen Lösungsmittelreste bei 0,001 mbar abgezogen. Es verblieben 4,26 g (96 % der Theorie) an (R)-2-(N-Benzyl-N-methyl)-aminopropionsäureethyl- ester in analysenreiner Form.

### Elementaranalyse:

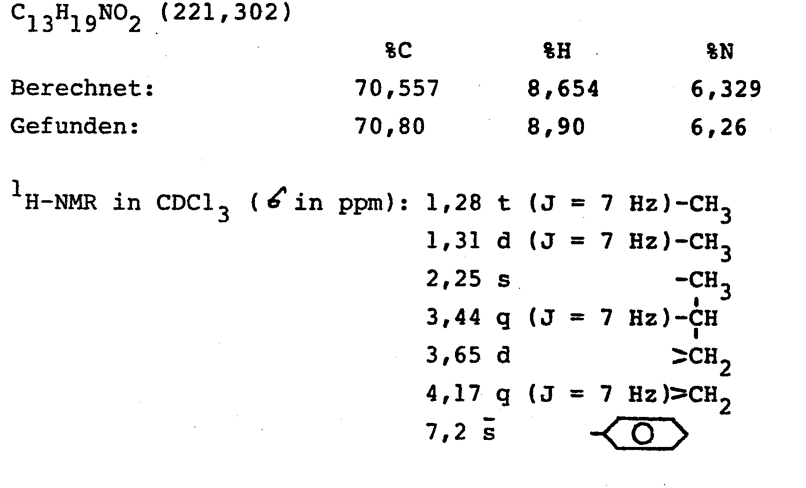

### Beispiel 4:

Zu 0,8 g (7,0 mmol) 1,6-Hexamethylendiamin in 25 ml _{D}i-chlormethan wurde bei 0 °C innerhalb von 20 Minuten eine Lösung von 1,75 g (7,0 mmol) (S)-O-Trifluormethylsulfonyl- milchsäureethylester in 25 ml Dichlormethan zugetropft. Nach 2,5-stündigem Nachrühren bei 20 °C wurden die ausgefallenen Salze abfiltriert, das Filtrat mit Wasser gewaschen und über Natriumsulfat getrocknet, das Lösungsmittel abdestilliert und der Rückstand unter vermindertem Druck fraktioniert destilliert. Es wurden 63,8 % der Theorie an (R,R)-Hexamethylen-1,6-bis-(2-aminopropionsäureethylester) erhalten, die bei 128 °C/0,065 mbar übergingen.

### Elementaranalyse:

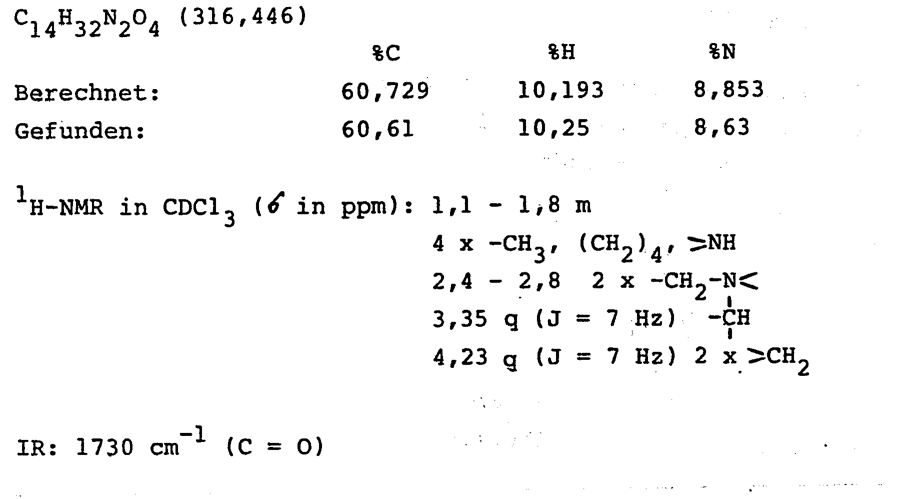

### Beispiele 5 bis 25:

Zu jeweils 14,0 mmol der angegebenen Aminoverbindung in 25 ml Dichlormethan wurde bei der jeweils angegebenen Temperatur innerhalb von 20 Minuten eine Lösung von 7,0 mmol (1,75 g) (S)-O-Trifluormethylsulfonyl-milchsäureethylester zugetropft. Es wurde die jeweils angegebene Zeit bei 25 °C nachgerührt. Dann wurden die ausgefallenen Salze abfiltriert, das Filtrat wurde mit Wasser gewaschen und über Natriumsulfat getrocknet, das Lösungsmittel abdestilliert und der Rückstand unter vermindertem Druck fraktioniert, umkristallisiert oder über eine Kieselgelsäule chromatographiert.

### Beispiel 5:

Eingesetzte Aminoverbindung: Anilin Reaktionstemperatur: 20 °C

Nachrührzeit: 6 Stunden

Produkt:

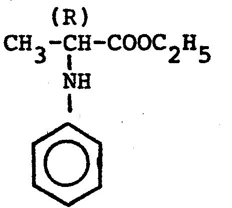

Siedepunkt: 70 °C/0,006 mbar Ausbeute: 75,9 % der Theorie

### Elementaranalyse:

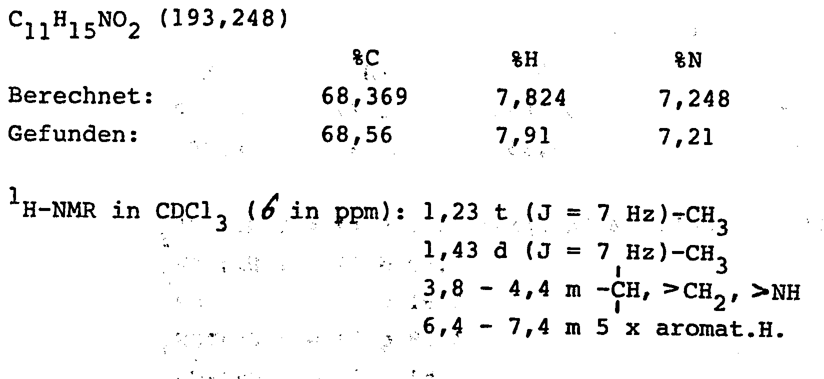

### Beispiel 6:

Eingesetzte Aminoverbindung: N-Methylanilin Reaktionstemperatur: 20 °C

### Nachrührzeit: 11 Stunden

### Produkt:

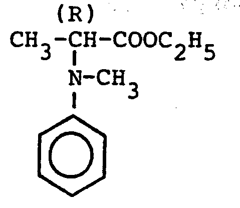

Siedepunkt: 78 °C/0,013 mbar Ausbeute: 74,9 % der Theorie

### Elementaranalyse:

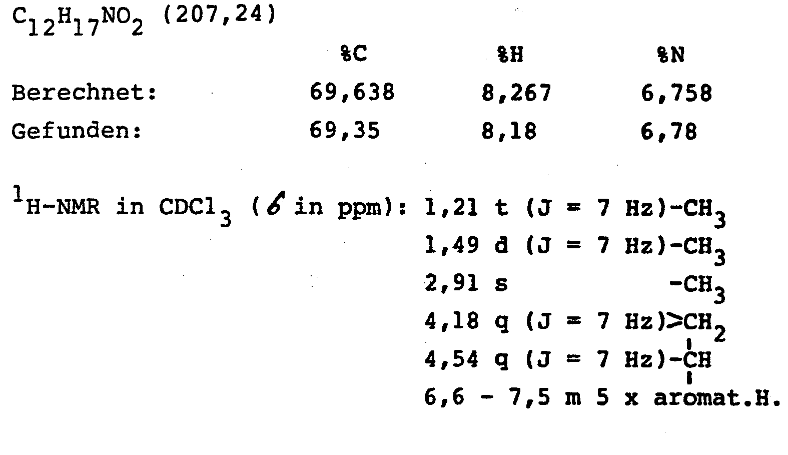

### Beispiel 7:

Eingesetzte Aminoverbindung: 3-Methoxyanilin Reaktionstemperatur: 20 °C

### Nachrührzeit: 14 Stunden

Produkt:

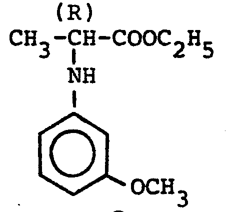

Siedepunkt: 103 °C/0,013 mbar Ausbeute: 89,6 % der Theorie

### Elementaranalyse:

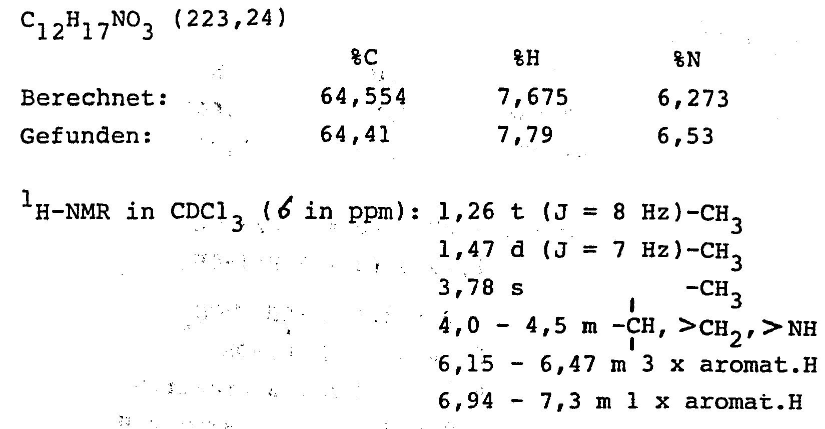

### Beispiel 8:

Eingesetzte Aminoverbindung: 3-Hydroxyanilin Reaktionstemperatur: 20 °C

Nachrührzeit: 20 Stunden

### Produkt

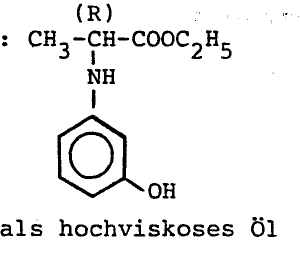

Ausbeute: 86,0 % der Theorie

### Elementaranalyse:

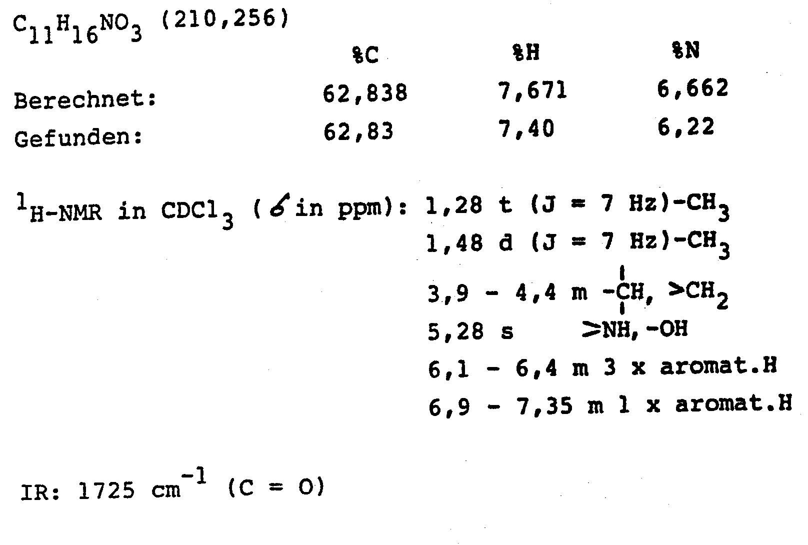

### Beispiel 9:

Eingesetzte Aminoverbindung: Benzylamin Reaktionstemperatur: 0 °C Nachrührzeit: 6 Stunden

### Produkt:

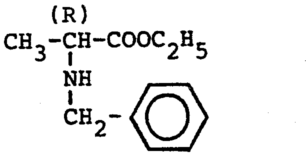

Siedepunkt: 72 °C/0,006 mbar Ausbeute: 92,6 % der Theorie

### Elementaranalyse:

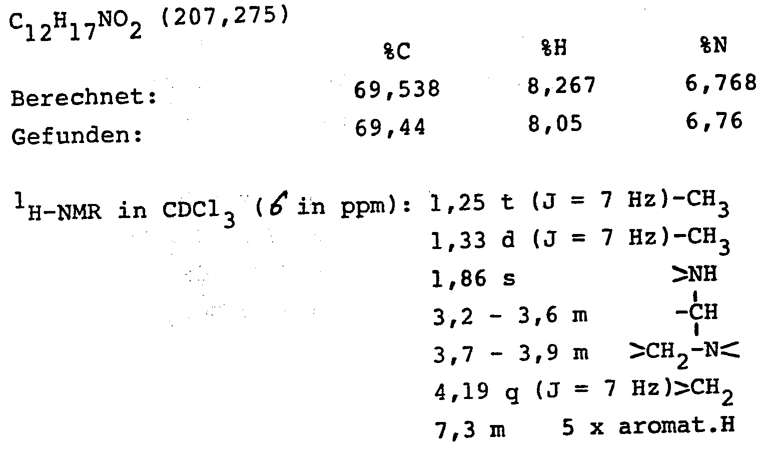

### Beispiel 10:

Eingesetzte Aminoverbindung: Diethylamin Reaktionstemperatur: 0 °C Nachrührzeit: 6 Stunden

### Produkt:

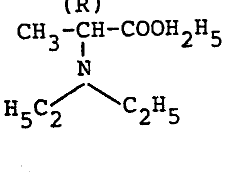

Siedepunkt: 62 °C/16 mbar Ausbeute: 74,2 % der Theorie

### Elementaranalyse:

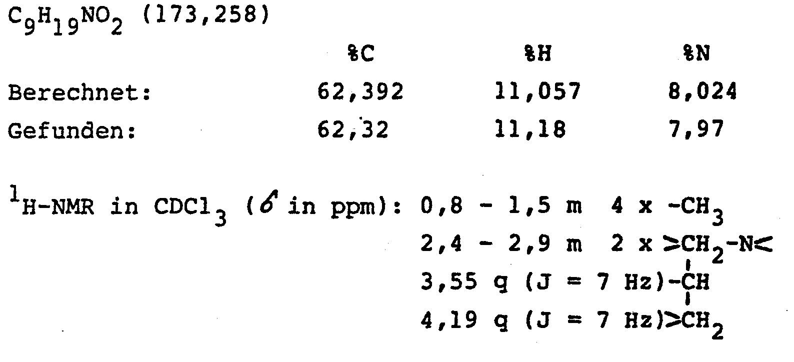

### Beispiel 11:

Eingesetzte Aminoverbindung: Pyrrolidin Reaktionstemperatur: 0 °C Nachrührzeit: 6 Stunden

### Produkt:

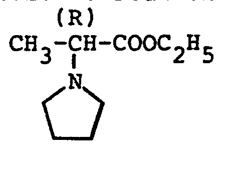

Siedepunkt: 73 °C/15 mbar Ausbeute: 94,8 % der Theorie

### Elementaranalyse:

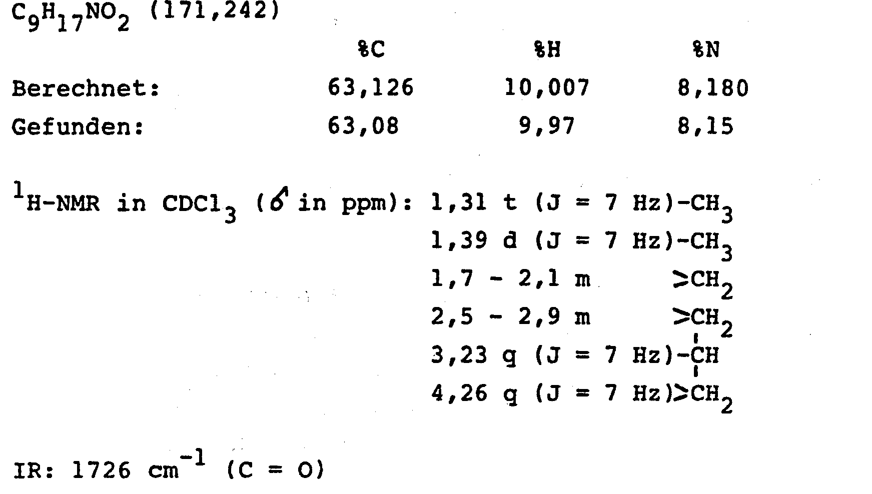

### Beispiel 12:

Eingesetzte Aminoverbindung: 6-Aminocapronsäuremethylester Reaktionstemperatur: 0 °C

Nachrührzeit: 6 Stunden

Produkt:

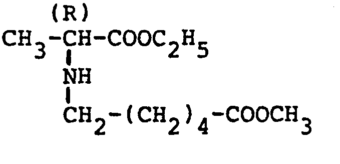

Ausbeute: 92,1 % der Theorie

### Elementaranalyse:

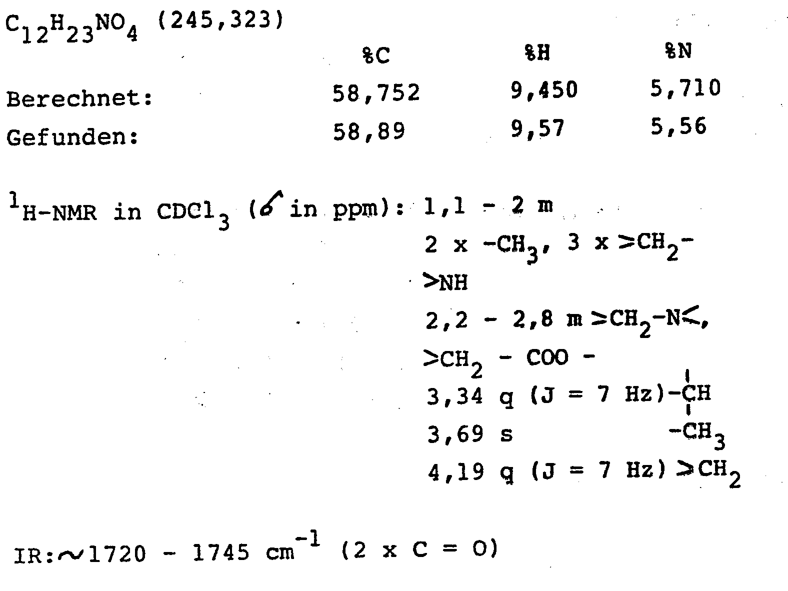

### Beispiel 13:

Eingesetzte Aminoverbindung: Monoethanolamin Reaktionstemperatur: 0 °C

Nachrührzeit: 6 Stunden

Produkt:

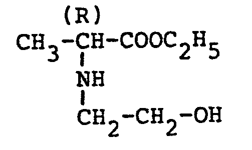

Siedepunkt: 60 °C/0,013 mbar Ausbeute: 60,8 % der Theorie

### Elementaranalyse:

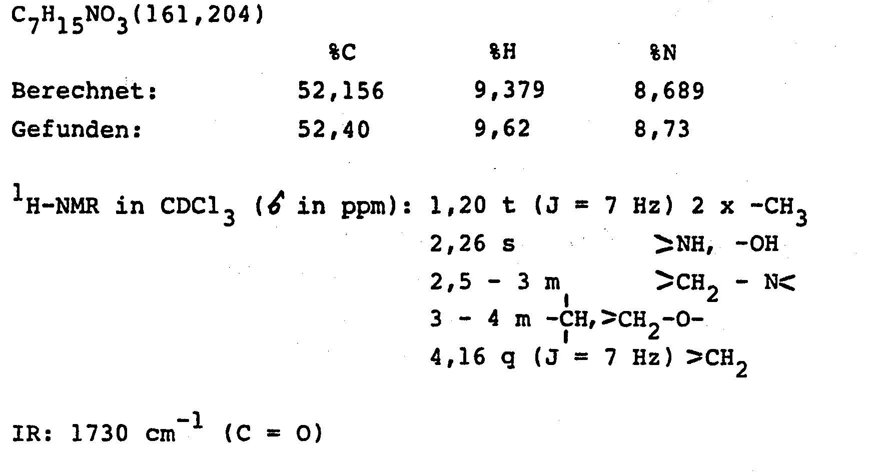

### Beispiel 14:

Eingesetzte Aminoverbindung: 4-Chloranilin Reaktionstemperatur: 20 °C

Nachrührzeit: 23 Stunden

### Produkt:

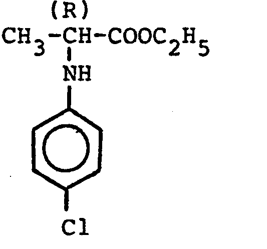

Siedepunkt: 90 - 91 °C/0,013 mbar Ausbeute: 79,5 % der Theorie

### Elementaranalyse:

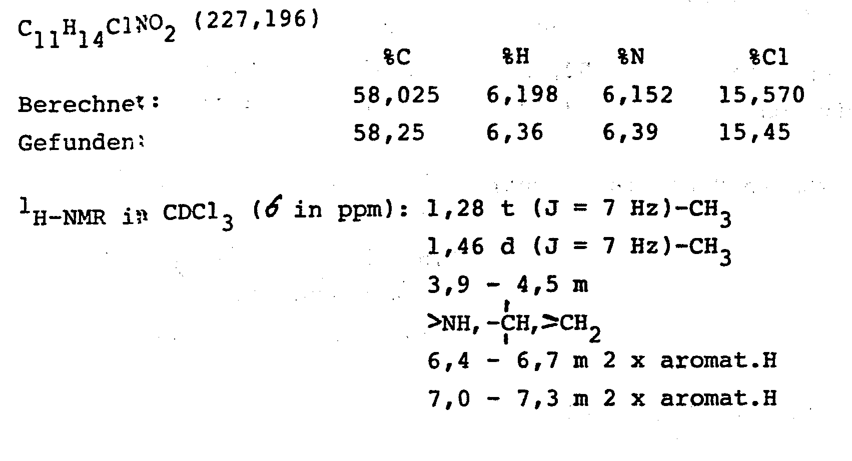

### Beispiel 15:

Eingesetzte Aminoverbindung: 3,4-Dichloranilin Reaktionstemperatur: 20 °C

Nachrühzeit: 30 Stunden Produkt:

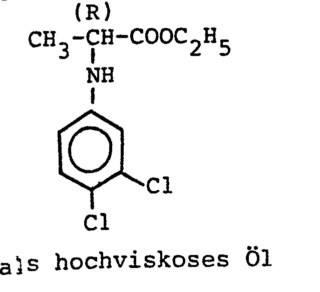

Ausbeute 92,3 % der Theorie

### Elementaranalyse:

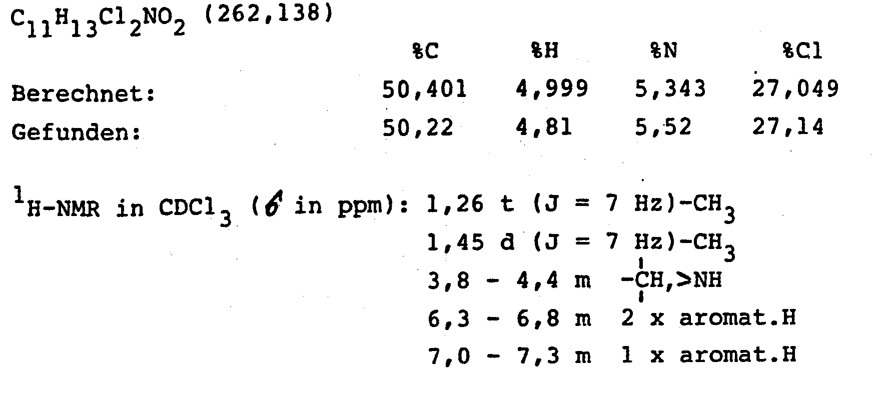

### Beispiel 16:

Eingesetzte Aminoverbindung: 2-Chloranilin Reaktionstemperatur: 20 °C

### Nachrührzeit: 52 Stunden

### Produkt:

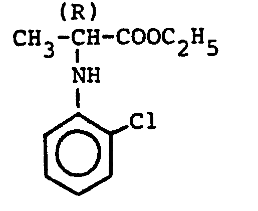

Siedepunkt: 72 °C/0,013 mbar Ausbeute: 83,0 % der Theorie

### Elementaranalyse:

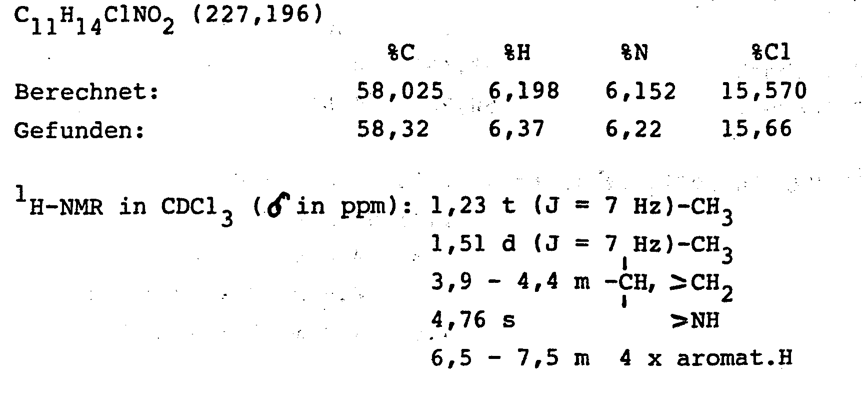

### Beispiel 17:

Eingesetzte Aminoverbindung: α-Naphthylamin Reaktionstemperatur: 20 °C Nachrührzeit: 24 Stunden

### Produkt:

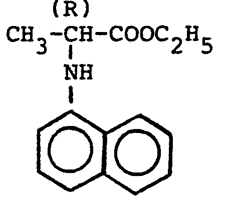

Siedepunkt: 128 °C/0,013 mbar Ausbeute: 75,2 % der Theorie

### Elementaranalyse:

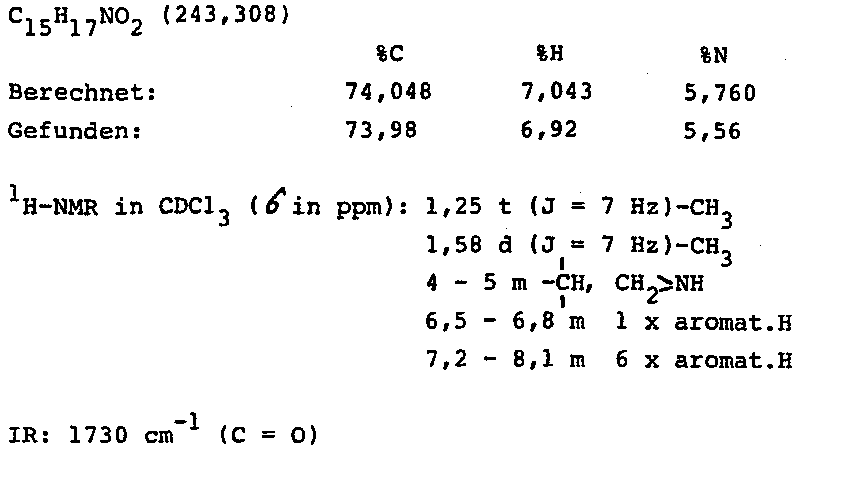

### Beispiel 18:

Eingesetzte Aminoverbindung: n-Hexylamin Reaktionstemperatur: 0 °C Nachrührzeit: 6 Stunden

### Produkt:

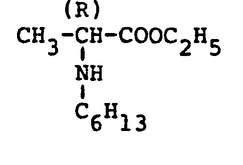

Siedepunkt: 42 °C/0,007 mbar Ausbeute: 90,2 % der Theorie

### Elementaranalyse:

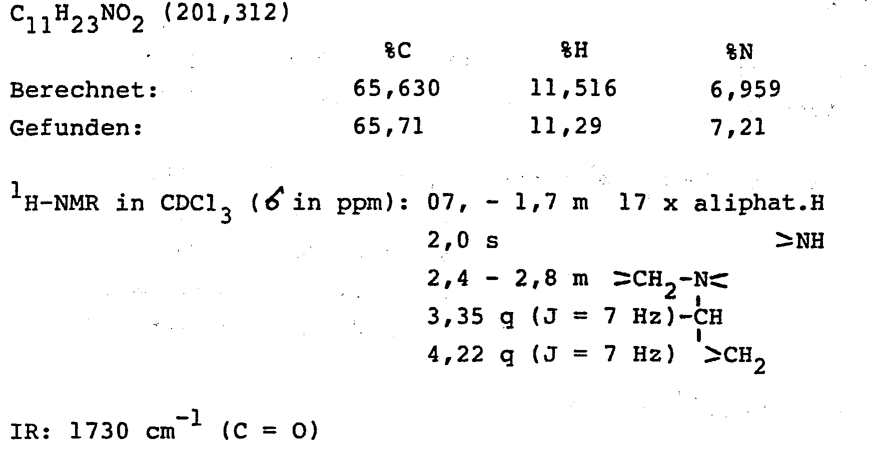

### Beispiel 19:

Eingesetzte Aminoverbindung: tert. Butylamin Reaktionstemperatur: 0 °C

Nachrührzeit: 6 Stunden

### Produkt:

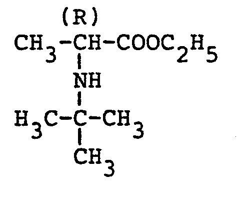

Siedepunkt: 56 °C/16 mbar Ausbeute: 77,9 % der Theorie

### Elementaranalyse:

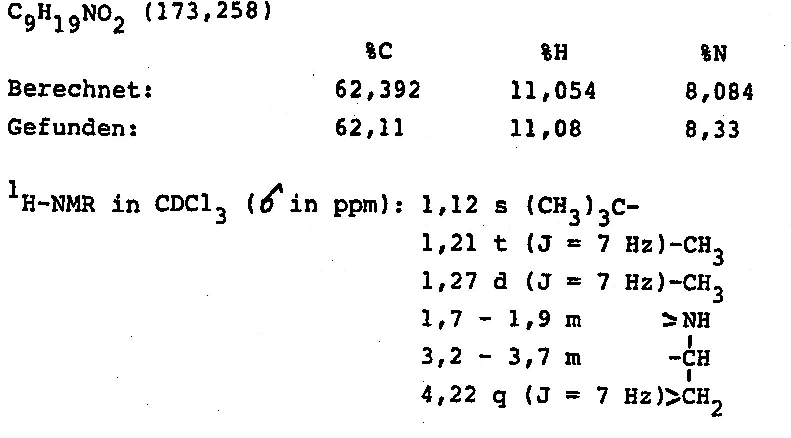

### Beispiel 20:

Eingesetzte Aminoverbindung: (R,S)-2-Ethylpiperidin Reaktionstemperatur: 0 °C Nachrührzeit: 6 Stunden

### Produkt:

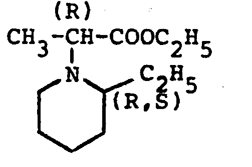

Siedepunkt: 47 - 48 °C/0,013 mbar Ausbeute: 85,4 % der Theorie

### Elementaranalyse:

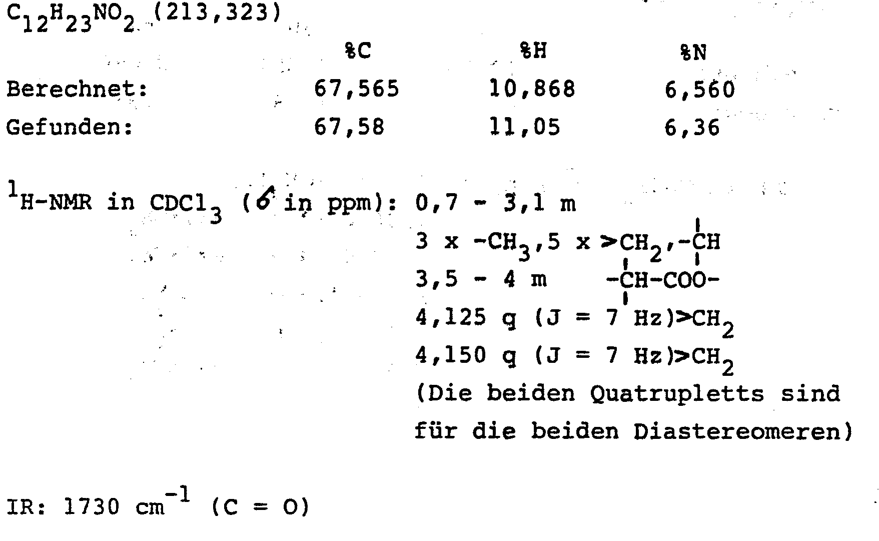

### Beispiel 21:

Eingesetzte Aminoverbindung: (R,S)-2-Aminobutan Reaktionstemperatur: 0°C

Nachrührzeit: 6 Stunden Produkt:

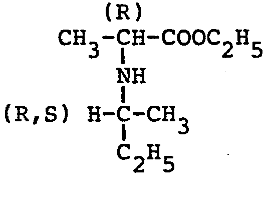

Siedepunkt: 62 °C/23 mbar Ausbeute: 67,6 % der Theorie

### Elementaranalyse:

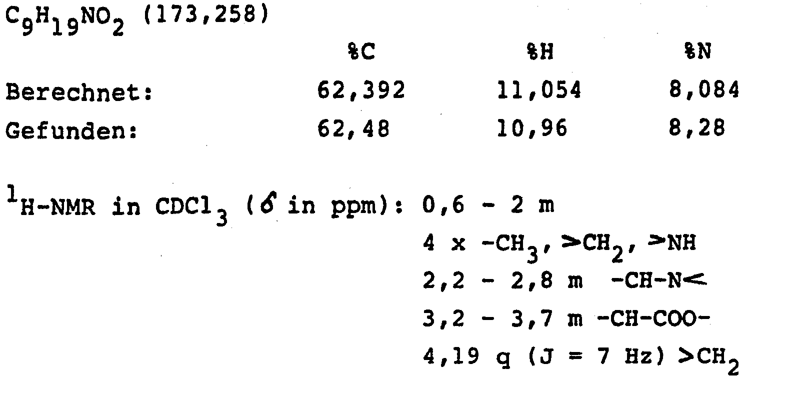

### Beispiel 22:

Eingesetzte Aminoverbindung: (R)-Prolinmethylester Reaktionstemperatur: 0 °C

Nachrührzeit: 2 Stunden

### Produkt:

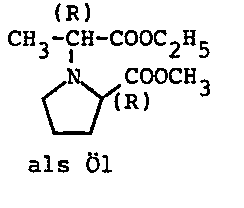

Ausbeute: 93,9 % der Theorie

### Elementaranalyse:

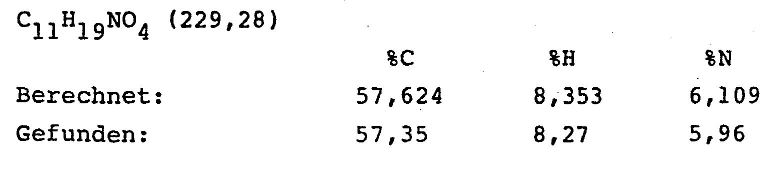

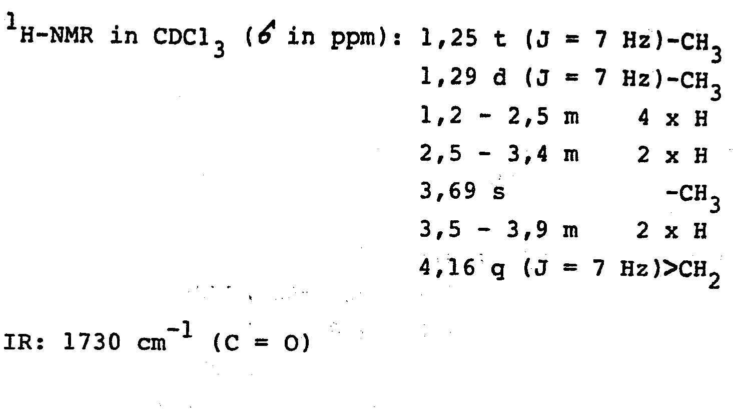

### Beispiel 23:

Eingesetzte Aminoverbindung: (S)-Prolinmethylester Reaktionstemperatur: 0 °C

Nachrührzeit: 2 Stunden

### Produkt:

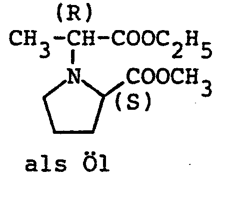

Ausbeute: 92,2 % der Theorie

### Elementaranalyse:

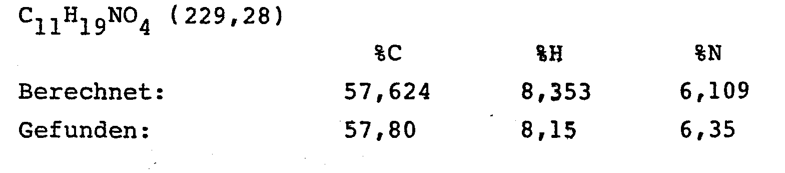

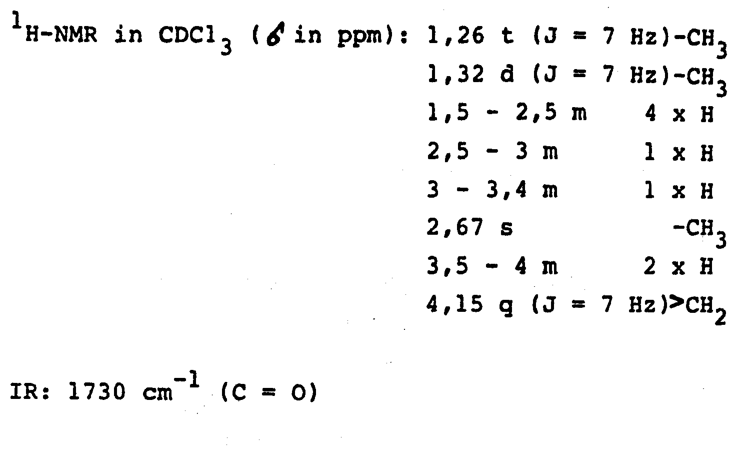

### Beispiel 24:

Eingesetzte Aminoverbindung: (R)-Phenylalaninmethylester Reaktionstemperatur: 0 °C

Nachrührzeit: 20 Stunden

### Produkt:

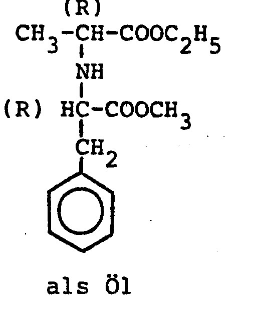

Ausbeute: 86,2 % der Theorie

### Elementaranalyse:

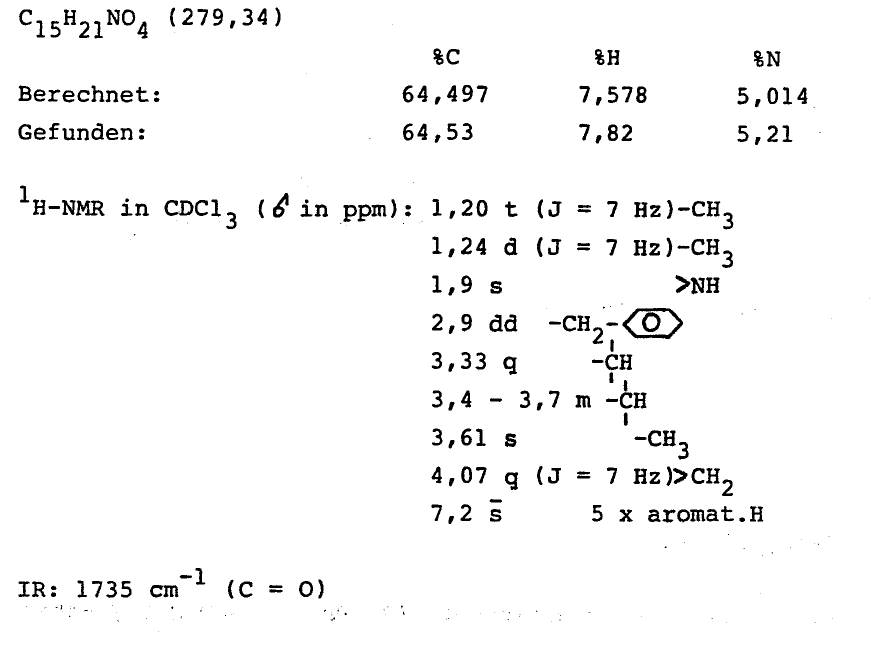

### Beispiel 25:

Eingesetzte Aminoverbindung: (S)-Phenylalaninmethylester Reaktionstemperatur: 0 °C

Nachrührzeit: 20 Stunden

### Produkt:

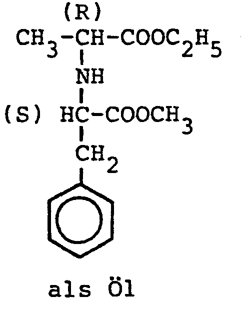

Ausbeute: 92,1 % der Theorie

### Elementaranalyse:

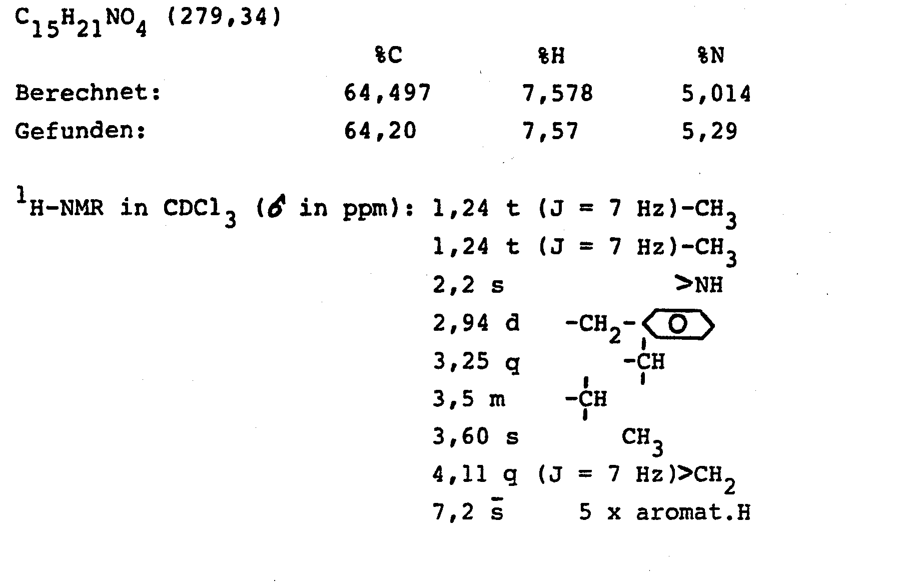

### Beispiel 26:

Zu 2,26 g (7,0 mmol) (S)-O-Trifluormethylsulfonyl-äpfel- säurediethylester in 25 ml Dichlormethan wurde bei 0 °C innerhalb von 20 Minuten eine Lösung von 1,30 g (14,0 mmol) Anilin in 25 ml Dichlormethan zugetropft. Nach 15 Stunden Nachrühren bei 25 °C wurden die ungelösten Salze abfiltriert und das Lösungsmittel wurde unter vermindertem Druck abdestilliert. Der hinterbleibende Rückstand wurde aus n-Pentan umkristallisiert. Es wurden 1,74 g (94 % der Theorie) an (R)-N-Phenylasparaginsäurediethylester mit einem Schmelzpunkt von 61 °C erhalten.

### Elementaranalyse:

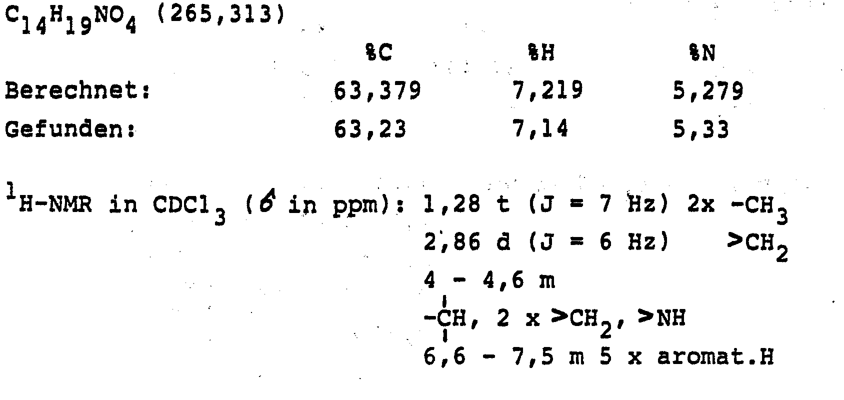

### Beispiel 27:

Zu 1,61 g (5,0 mmol) (S)-O-Trifluormethylsulfonyl-äpfel- säurediethylester in 20 ml Dichlormethan wurde bei 0 °C innerhalb von 20 Minuten eine Lösung von 1,63 g (10,0 mmol) 3,4-Dichloranilin in 20 ml Dichlormethan zugetropft. Nach 45-stündigem Nachrühren bei 25 °C wurde das Lösungsmittel abdestilliert und der Rückstand in einem Gemisch aus n-Pentan und Diethylether (1 : 1, v/v) aufgenommen. Die ungelösten Salze wurden abfiltriert und die Lösungsmittel unter vermindertem Druck abdestilliert. Es verblieben 1,48 g (89 % der Theorie) an (R)-N-(3,4-Dichlorphenyl)-asparagin- säurediethylester als Öl.

### Elementaranalyse:

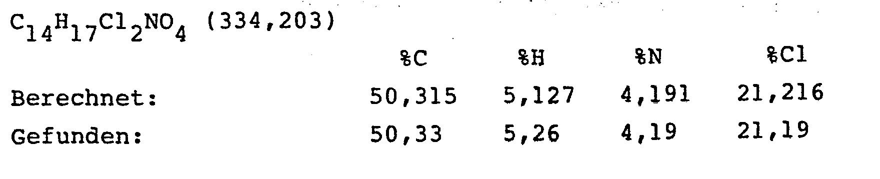

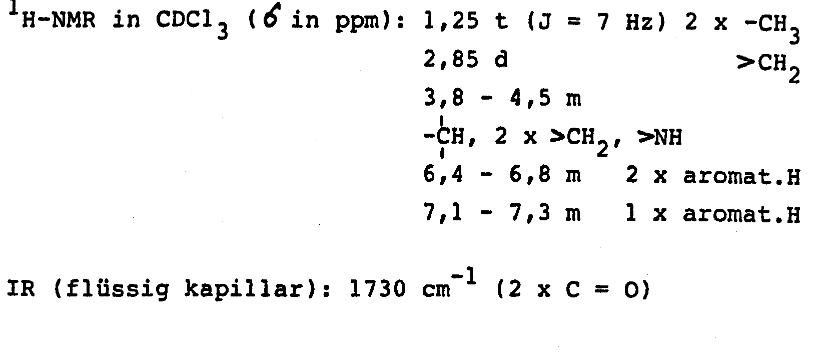

### Beispiel 28:

Zu 2,26 g (7,0 mmol) (S)-0-Trifluormethylsulfonyl-äpfel- säurediethylester in 25 ml Dichlormethan wurde bei -70 °C innerhalb von 1,25 Stunden eine Lösung von 1,50 g (14,0 mmol) Benzylamin in 25 ml Dichlormethan zugetropft. Nach Erwärmen auf 25 °C wurde das Reaktionsgemisch mit Wasser gewaschen, die organische Phase abgetrennt und über Natriumsulfat getrocknet und das Lösungsmittel unter vermindertem Druck abdestilliert. Der Rückstand wurde im Hochvakuum von letzten Lösungsmittelresten befreit. Es verblieben 1,82 g (93 % der Theorie) an gaschromatographisch reinem (R)-N-Benzyl-asparaginsäurediethylester, der sich bei 113 °C/0,013 mbar destillieren ließ.

### Elementaranalyse:

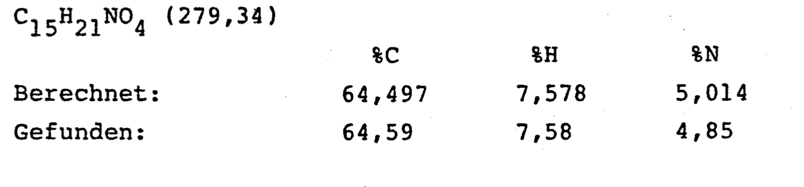

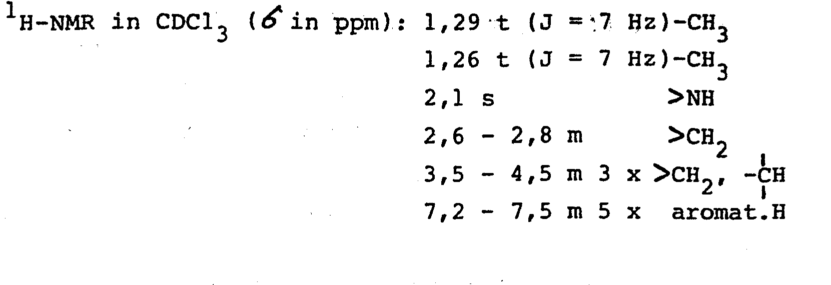

### Beispiel 29:

Zu 937 mg (3,0 mmol) (S)-0-Trifluormethylsulfonyl-phenyl- milchsäuremethylester in 10 ml Dichlormethan wurde bei 0 °C innerhalb von 20 Minuten eine Lösung von 727 mg (6,0 mmol) (S)-Phenethylamin in 10 ml Dichlormethan zugetropft. Nach 15-stündigem Nachrühren bei 25 °C wurden die ausgefallenen Salze abfiltriert. Dann wurde die organische Phase mit Wasser gewaschen, abgetrennt und über Natriumsulfat getrocknet. Das Lösungsmittel wurde unter vermindertem Druck abdestilliert und der Rückstand über eine 20 cm hohe Kieselgel-Säule mit einem Laufmittelgemisch aus Petrolether und Essigsäureethylester (3 : 1, v/v) chromatographiert. Nach dem Eindampfen des Eluats verblieben 785 mg (92 % der Theorie) an N- [(S)-Methylbenzyl]-(R)-phenylalaninmethyl- ester in analysenreiner Form.

### Elementaranalyse:

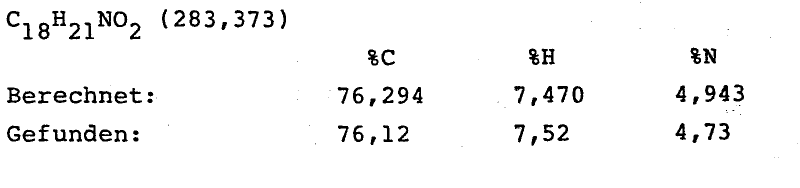

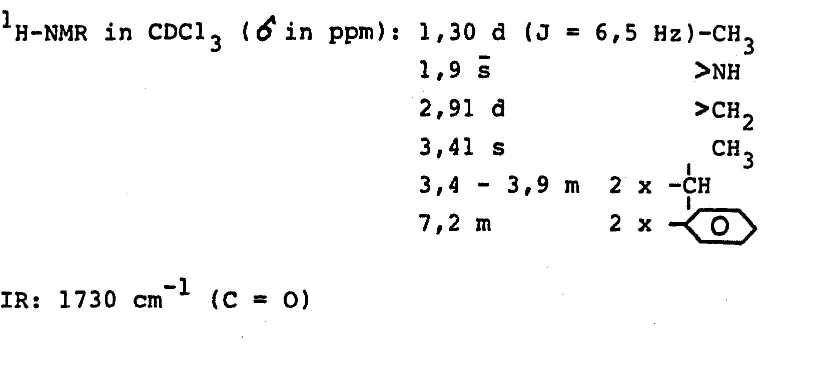

### Beispiel 30:

Zu 2,19 g (7,0 mmol) (S)-0-Trifluormethylsulfonyl-phenyl- milchsäuremethylester in 25 ml Dichlormethan wurde bei 0 °C innerhalb von 20 Minuten eine Lösung von 1,50 g (14,0 mmol) Benzylamin in 25 ml Dichlormethan zugetropft. Es wurde auf 25 °C erwärmen lassen und nach 2 Stunden wurden die ausgefallenen Salze abfiltriert. Das Lösungsmittel wurde abdestilliert und der Rückstand in n-Pentan aufgenommen. Nach nochmaligem Filtrieren wurde das n-Pentan abdestilliert und der Rückstand bei 0,001 mbar von Lösungsmittelresten befreit. Es verblieben 1,74 g (92 % der Theorie) an (R)-N-Benzyl-phenylalaninmethylester in analysenreiner Form.

### Elementaranalyse:

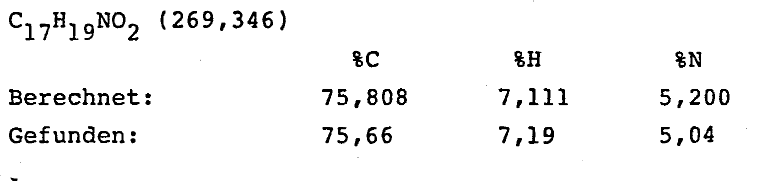

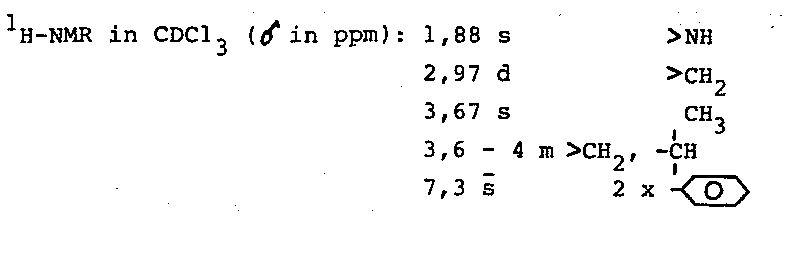

### Beispiel 31:

Zu 2,19 g (7,0 mmol) (S)-O-Trifluormethylsulfonyl-phenyl- milchsäuremethylester in 25 ml Dichlormethan wurde bei 0 °C innerhalb von 20 Minuten eine Lösung von 1,30 g (14,0 mmol) Anilin in 25 ml Dichlormethan zugetropft. Es wurde auf 25 °C erwärmen lassen und nach 2 Stunden wurden die ausgefallenen Salze abfiltriert. Das Lösungsmittel wurde abdestilliert und der Rückstand unter Verwendung von Dichlormethan als Laufmittel über eine 20 cm hohe Kieselgelsäule chromatographiert. Nach dem Eindampfen des Eluats und Entfernung der Lösungsmittelreste im Hochvakuum verblieben 1,66 g (92 % der Theorie) an analysenreinem (R)-N-Phenyl- phenylalaninmethylester.

### Elementaranalyse:

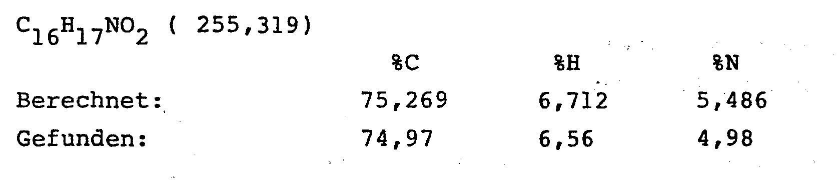

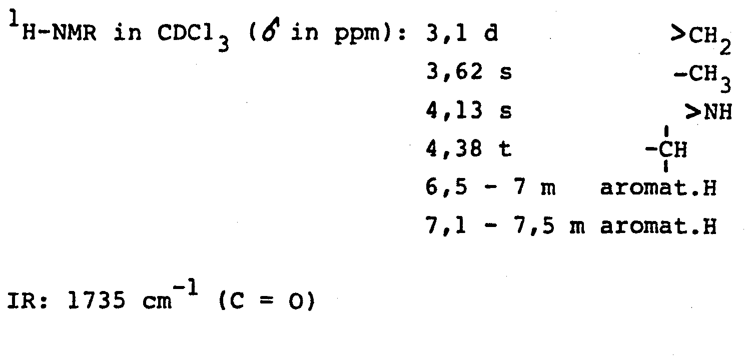

### Beispiel 32:

Zu 1339 mg (3,0 mmol) (S)-0-Trifluormethylsulfonyl-äpfel- säuredibenzylester in 25 ml Dichlormethan wurde bei -70 °C innerhalb von 40 Minuten eine Lösung von 643 mg (6,0 mmol) Benzylamin in 25 ml Dichlormethan zugetropft. Es wurde über Nacht nachgerührt und nach Erwärmen auf 20 °C wurden die ausgefallenen Salze abfiltriert. Das Filtrat wurde mit Wasser gewaschen, über Natriumsulfat getrocknet und eingeengt. Der Rückstand wurde über eine 30 cm hohe Kieselgel- säule chromatographiert. Mit Dichlormethan als Laufmittel wurden zunächst 190 mg Fumarsäuredibenzylester (Schmelzpunkt 57 bis 59 °C), mit einem Gemisch aus Petrolether und Essigsäureethylester (3 : 1, v/v) als Laufmittel dann 684 mg (57 % der Theorie) (R)-N-Benzyl-asparaginsäurediben- zylester als analysenreines hochviskoses Öl eluiert.

### Elementaranalyse:

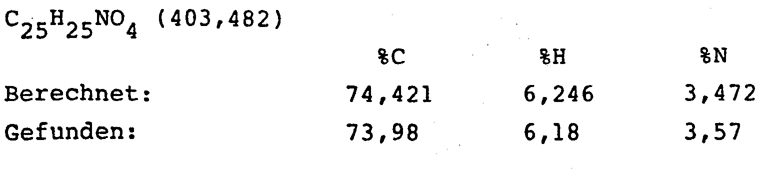

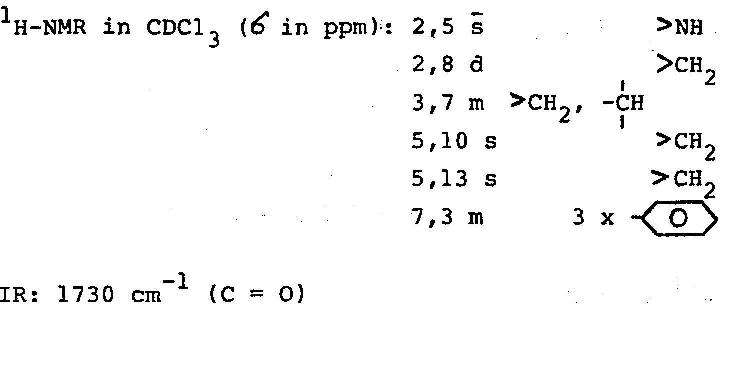

### Beispiel 33:

Zu 5,80 g (13,0 mmol) (S)-0-Trifluormethylsulfonyl-äpfel- säuredibenzylester in 35 ml Dichlormethan wurde bei -10 °C innerhalb von 20 Minuten eine Lösung von 2,42 g (26,0 mmol) Anilin in 35 ml Dichlormethan zugetropft. Es wurde noch eine Stunde nachgerührt und unter Erwärmen auf 20 °C über Nacht stehen gelassen. Die ausgefallenen Salze wurden abgesaugt und das Filtrat eingeengt. Der Rückstand wurde aus Methanol umkristallisiert. Es wurden 4,67 g (92 % der Theorie) an (R)-N-Phenyl-aspäraginsäuredibenzylester mit einem Schmelzpunkt von 95 bis 96 °C erhalten.

### Elementaranalyse:

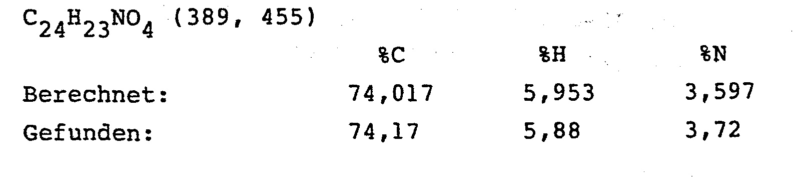

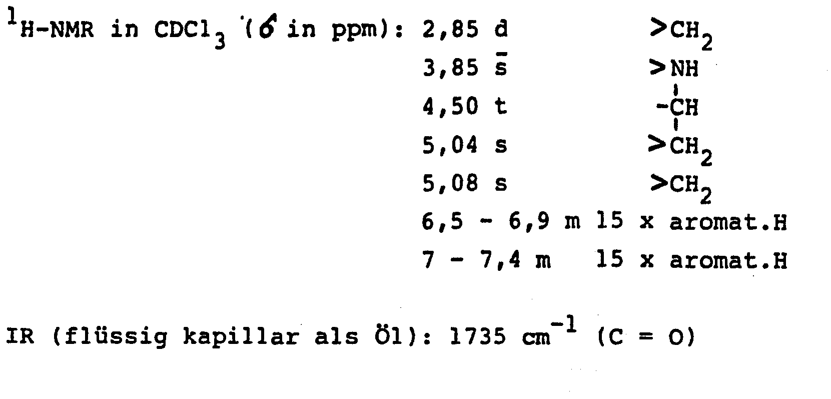

### Beispiel 34:

Entsprechend Beispiel 33 wurden aus 2,68 g (6,0 mmol) (R)-0-Trifluormethylsulfonyl-äpfelsäuredibenzylester in 20 ml Dichlormethan und 1,12 g (12 mmol) Anilin in 20 ml Dichlormethan 2,18 g (93 % der Theorie) an (S)-N-Phenyl-asparagin- säuredibenzylester mit einem Schmelzpunkt von 96 °C erhalten.

## Patentansprüche

1. Verfahren zur praktisch stereospezifischen Herstellung von optisch aktiven α-Aminocarbonsäureestern der allgemeinen Formel 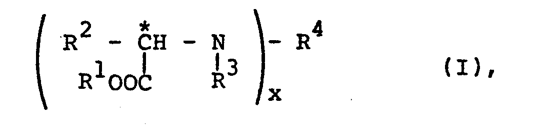 in der
^{*} ein Asymmetriezentrum,
x die Zahl 1 oder 2,
R¹ einen Methyl-, Ethyl- oder Benzylrest,
R² einen geradkettigen oder verzweigten, unsubstituierten oder ein- oder mehrfach und in beliebiger Stellung durch Fluor, Chlor, Brom, eine (C₁ bis C₈)-Alkoxygruppe, eine Methoxy-, Ethoxy- oder Benzoxycarbonylgruppe oder einen unsubstituierten oder 1,2 oder 3 Substituenten tragenden Phenylrest substituierten (C1 bis C₈)-Alkylrest oder einen unsubstituierten oder 1,2 oder 3 _{S}ub- stituenten tragenden Phenylrest und
_{R}³ Wasserstoff, einen geradkettigen oder verzweigten, unsubstituierten oder ein- oder mehrfach und in beliebiger Stellung durch Fluor, Chlor, Brom, eine Cyanogruppe, eine Hydroxylgruppe, eine (C₁ bis C₈)-Alkoxygruppe, eine Methoxy-, Ethoxy- oder Benzoxycarbonylgruppe, eine Di-(C₁ bis C₈)-alkylaminogruppe, einen unsubstituierten oder 1,2 oder 3 Substituenten tragenden Phenylrest oder einen unsubstituierten oder 1 bis 6 Substituenten tragenden Naphthylrest substituierten (C₁ bis C₁₂)-Alkylrest oder einen unsubstituierten oder 1,2 oder 3 Substituenten tragenden Phenylrest oder einen unsubstituierten oder 1 bis 6 Substituenten tragenden Naphthylrest bedeuten,
für x = 1 _{R}⁴ unabhängig von R³ mit Ausnahme von Wasserstoff eine der für R³ angegebenen Bedeutung hat oder zusammen mit R³ und dem Stickstoffatom einen gesättigten oder ungesättigten Ring mit 4 bis 7 Ringgliedern bildet, der 0 bis 2 weitere Heteroatome aus der Gruppe Stickstoff, Schwefel oder Sauerstoff aufweist und unsubstituiert oder ein- oder mehrfach durch die oben definieten Substituenten R² oder R³ substituiert ist, oder
für x = 2 R⁴ einen geradkettigen oder verzweigten Alkylenrest mit 1 bis 12 Kohlenstoffatomen darstellt, dadurch gekennzeichnet, daß man einen 0-Perfluoralkylsulfonyl-hydroxycarbonsäureester der allgemeinen Formel 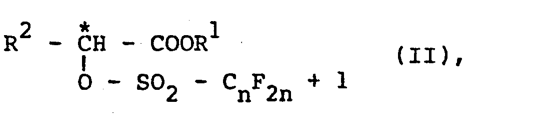 in der ∗, _{R}¹ und R² wieder die bereits angegebene Bedeutung haben und n eine ganze Zahl von 1 bis 6 bedeutet, in einem inerten Lösungsmittel bei einer Temperatur zwischen -80 und +50 °C mit einer Aminoverbindung der allgemeinen Formel 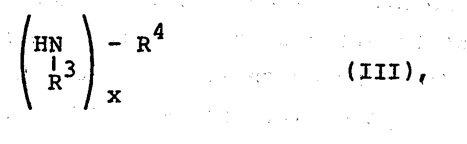 in der x, R³ und _{R4} wieder die bereits angegebene Bedeutung haben, umsetzt, wobei die Aminoverbindung der allgemeinen Formel (III), jeweils bezogen auf den eingesetzten 0-_{P}erfluoralkylsulfonyl-hydroxycarbonsäureester der allgemeinen Formel (II), für x = 1 in der 2- bis 2,5-fachen Molmenge und für x = 2 in der 1- bis 1,25- fachen Molmenge eingesetzt wird.

2. Verfahren nach Anspruch 1, dadurch gekennzeichnet, daß man die Umsetzung zwischen dem 0-Perfluoralkylsulfonyl-hydroxycarbonsäureester der allgemeinen Formel (II) mit der Aminoverbindung der allgemeinen Formel (III) bei einer Temperatur zwischen -40 und +25 °C vornimmt.
